Europäisches Patentamt

(19) European Patent Office

Office européen des brevets

(11) Publication number : **0 432 835 B1**

# (12) EUROPEAN PATENT SPECIFICATION

(45) Date of publication of patent specification :
02.03.94 Bulletin 94/09

(51) Int. Cl.⁵ : **A23L 1/05, A23D 7/00, // A61K7/00**

(21) Application number : **90203209.3**

(22) Date of filing : **06.12.90**

(54) **Fluid composition.**

(30) Priority : **15.12.89 GB 8928370**

(43) Date of publication of application :
**19.06.91 Bulletin 91/25**

(45) Publication of the grant of the patent :
**02.03.94 Bulletin 94/09**

(84) Designated Contracting States :
**AT BE CH DE DK ES FR GB GR IT LI NL SE**

(56) References cited :
EP-A- 0 271 132
EP-A- 0 355 908
WO-A-89/12403
JP-A-61 288 396
US-A- 2 859 115
US-A- 3 764 707
US-A- 4 305 970

(73) Proprietor : **UNILEVER N.V.**
**Weena 455**
**NL-3013 AL Rotterdam (NL)**
(84) **BE CH DE DK ES FR GR IT LI NL SE AT**
Proprietor : **UNILEVER PLC**
**Unilever House Blackfriars P.O. Box 68**
**London EC4P 4BQ (GB)**
(84) **GB**

(72) Inventor : **Hedges, Nicholas David, Unilever**
**Research Labor.**
**Colworth, Colworth House**
**Sharnbrook, Bedford, MK44 1LQ (GB)**
Inventor : **Norton, Ian Timothy, Unilever**
**Research Laboratory**
**Colworth, Colworth House**
**Sharnbrook, Bedford, MK44 1LQ (GB)**

(74) Representative : **Mulder, Cornelis Willem**
**Reinier, Dr. et al**
**UNILEVER N.V. Patent Division P.O. Box 137**
**NL-3130 AC Vlaardingen (NL)**

EP 0 432 835 B1

## Description

The present invention is concerned with a process for preparing a liquid based composition comprising at least one chemically setting gelling agent. Here by a chemically setting gelling agent is meant a component which, after being dispersed into a liquid, will set to a gel when allowed to chemically interact with a supplementary active component, which active component usually is a cation. Examples of such chemically setting gelling agents are: alginate, pectin, iota-carrageenan, kappa-carrageenan and furcellaran.

The process of chemically setting gelling agents of the above type is well known in the art. The processes described in the art have in common that the gel setting process is effected under relatively quiescent conditions, resulting in a rigid gel. Such a rigid gel is suitable for many product applications such as puddings, jellies etc.

In WO 89/12403, which was published after the first filing date of the present application, a fat substitute is disclosed which comprises water-dispersible macrocolloid particles of carbohydrate materials which particles have a substantially spheroidal shape. In Examples 4 and 5 the chemical setting of sodium alginate is described by combining sodium alginate and calcium chloride and shearing in a fluid processor apparatus cooled with tap water.

We have found now that a fluid composition having various advantageous properties can be obtained by shearing a liquid containing a chemically setting gelling agent, while gelation is effected. Accordingly the present invention relates to a process for preparing a fluid composition containing a chemically setting gelling agent, wherein a liquid containing the gelling agent is chemically set, whilst subjecting said liquid to sufficient shear to obtain a substantially less rigid composition than would have been obtained by chemically setting the liquid under quiescent conditions, wherein the composition comprises at least 75 wt% of the liquid and wherein either (a) the gelling agent comprises a gelling agent selected from the group consisting to pectin, iota-carrageenan, kappa-carrageenan, furcellaran and mixtures thereof, or (b) the gelling agent is alginate and the liquid containing the gelling agent in set at a temperature above 30°C.

The fluid compositions according to the present invention possess favourable rheological properties which are particularly appreciated when these fluid compositions are incorporated in, for instance, skin creams, moisturizer lotions, spreads, etc. The smooth disruption at low strain of the present composition is essentially different from the fracturing observed when rigid gels are subjected to conditions of strain. The disruption at low strain (e.g. rubbing or mastication) imparts to the present composition a very smooth fat-like consistency. Due to its fat-like rheology the present fluid composition can suitably be used as a fat substitute. Accordingly the fluid composition can suitably be used to prepare 0% fat spreads or water-continuous low fat spreads.

Another advantage of the present fluid composition is that it can suitably be used in the preparation of fat-continuous spreads. The fluid composition according to the invention, comprising more or less discrete gelled particles, referred to in this document as microgels, can advantageously be dispersed in a fat matrix so as to obtain a stable water-in-oil dispersion. In this manner dispersions can be prepared that contain water-soluble ingredients which would hinder the preparation of a water-in-oil dispersion when using conventional processing.

The use of the fluid composition according to the invention in the preparation of water-in-oil emulsions such as spreads furthermore offers the advantage that the size of the gelled droplets in the emulsion depends heavily on the size of the microgels in the fluid composition. Thus the droplet size can be controlled more easily than in a conventional process wherein a non-gelled aqueous phase is dispersed into the fat phase.

As compared to the fluid compositions described in copending application EP-A 89 20 2066, which compositions contain gelling agents capable of forming reversible gels, the composition obtained by the present process offers the advantage that it is stable towards pasteurization conditions. Thus the present composition can be incorporated into a spread using conventional processing techniques. An additional advantage offered by the present composition is that variations in the cationic content and composition of the aqueous phase have little or no influence on its rheology.

The mobile condition of the fluid composition obtained by the present process should not be confused with the enhancement of mobility which can be achieved by breaking up a gel into particles which can roll and slide over one another. The gel particles thus obtained are of macroscopical size and are visible to the naked eye, i.e. they have an average size exceeding 100 micrometer. Thus effectively such a broken gel is composed of numerous relatively large fragments, possessing the same properties, e.g. rigidity, as the original gel. Another noticeable difference between broken gels and the compositions according to the invention is that broken gels display a clear tendency to loose liquid.

Although in principle any food grade chemically setting gelling agent may be used in the present process, preferably, said gelling agent is selected from the group consisting of alginate, pectin, iota-carrageenan, kappa-carrageenan, furcellaran. and mixtures thereof. More preferably the gelling agent used in the present process is selected from the group consisting of alginate, pectin and mixtures thereof. Most preferably the gelling agent used in the present process is pectin.

In the present process generally the liquid is chemically set by allowing the gelling agent to form a salt with an effective cation. The latter may be effected, for instance, by adding said effective cation to the liquid containing the gelling agent or alternatively by converting a precursor-compound, present in the liquid containing the gelling agent, into the effective cation. The cation utilized in the present process, preferably, is selected from the group consisting of $Ca^{2+}$, $K^+$ and mixtures thereof. Most preferably the cation is $Ca^{2+}$.

The present process can be carried out as a batch process, but it is preferred to carry out the process in a continuous or semi-continuous manner by, for instance, combining two separate streams, one containing the gelling agent, the other containing the effective cation. Thus, according to preferred embodiment, the liquid is chemically set by combining two separate streams, one containing the gelling agent and another containing the cation. In an even more preferred embodiment the two streams are separately fed to an apparatus in which both streams are combined whilst applying and maintaining sufficient shear to obtain a substantially less rigid composition than would have been obtained by chemically setting the liquid under quiescent conditions. The liquid containing the gelling agent is preferably chemically set at a temperature above 20°C, more preferably above 30°C.

As contrasted with broken gels, the fluid composition obtained by the present process, when viewed under a microscope, comprises very small microgels, which microgels, preferably, have a mean equivalent diameter not exceeding 100 micrometer, more preferably not exceeding 50 microns. Here the mean equivalent diameter is the number weighted mean equivalent diameter of the microgels having an equivalent diameter in the range of 0.1 to 200 micrometer. Most preferably the microgels present in the fluid composition obtained by the process of the invention have a mean equivalent diameter in the range of 0.1 to 30 micrometer. According to yet another preferred embodiment at least 2%, more preferably at least 10% of the number of microgels exceed 5 microns, using a Coulter Counter MultiSizer analysis as in WO 89/12403.

The equivalent diameter distribution of the microgels can suitably be established from microscopical images. Although such may be done by hand, in order to obtain more reproducible results, it is preferred to determine the diameter distribution by means of an image analyzing computer. Of course the magnification applied should be suitable for properly determining the diameter distribution. For determining the diameter distribution in the range of 0.1 to 100 micrometer a magnification of 800 appeared to be appropriate. Furthermore it should be observed that the mean equivalent diameter found, when using an image analyzing computer, may depend on the particle concentration, since overlapping particles may not be recognized as separate particles, but instead be regarded as one larger particle.

The size of the microgels obtained in the present process is largely determined by the intensity and duration of the shear employed. Preferably the gelling agent containing liquid is subjected to sufficient shear to obtain a fluid composition in which the gelling agent is predominantly present as microgels having a mean equivalent diameter of less than 100 micrometer, preferably of less than 50 micrometer.

Although the polysaccharide microgels obtained in the process of the invention can have a size of less than 0.1 micron or more than 200 micrometer, preferably, more than 90%, more preferably more than 95% by weight of the polysaccharides is present in the form of microgels having an equivalent diameter in the range of 0.1 to 200 micrometer, more preferably in the range of 0.1 to 100 micrometer.

According to a very preferred embodiment of the present invention the process comprises the additional step of drying the fluid composition obtained to a granular powder containing less than 50 wt.%, preferably less than 25 wt.% of the liquid. A pourable composition having essentially the same characteristics as the original fluid composition can be reconstituted by dispersing the powder into water. Due to the thermal stability of the structure formed during gelation, the powder can be contacted with hot water without any detrimental effects on the properties of the composition obtained. It is beneficial to indeed use hot water as otherwise it is difficult to adequately disperse the powder into the water.

The fluid composition can be dried by methods well known in the art. Preferably drying is effect to freeze drying, spray drying or flash drying.

An aspect of the present invention is a product obtainable by the above process. The product obtained by the process of the invention is characterized by the fact that it comprises microgels, which microgels, preferably, have a mean equivalent diameter not exceeding 100 micrometer, more preferably not exceeding 50 micrometer.

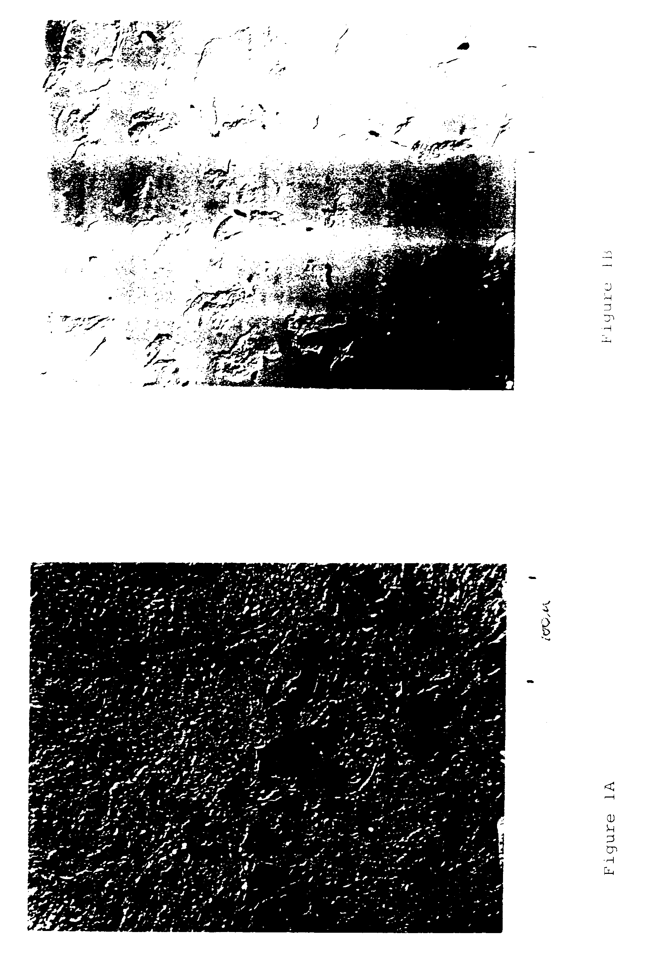

Although we do not wish to be bound by theory it is believed that the mobile state of the present fluid composition may be explained from the absence, on a macroscopical scale, of a three dimensional network. As can be seen from figures 1A and 1B, respectively representing a photograph of the microscopical image of a pourable composition according to the present invention and a micro-photograph of the same composition diluted with a factor 10, said pourable composition comprises essentially non-aggregated microgels. The microgels in the present composition may be of a spherical or irregular shape. Preferably essentially all the microgels present in the fluid composition are irregularly shaped, i.e. do not have a substantially spheroidal shape.

Another aspect of the present invention is a fluid composition containing a chemically setting gelling agent, at least part of which gelling agent has been chemically set, wherein the chemically set gelling agent is predominantly present as microgels having a mean equivalent diameter of less than 100 micrometer, preferably of less than 50 micrometer, and wherein either (a) the chemically setting gelling agent comprises a gelling agent selected from the group consisting of pectin, iota-carrageenan, kappa-carrageenan, furcellaran and mixtures thereof, and/or (b) the microgels are irregularly shaped.

The present fluid composition is preferably based on a polar liquid. More preferably said liquid is selected from the group consisting of: water, ethanol, isopropanol and mixtures thereof. For most purposes water is the most suitable liquid to be used. The present composition, at room temperature, comprises at least 75 wt.% of the liquid.

Whenever referred to in this document, by the liquid is meant that liquid phase in the product in which the gelling agent dissolves most easily. The concentration level of chemically setting gelling agent in the present composition can suitably lie below 8 % by weight of the liquid, but higher concentrations are possible. A more preferred range of gelling agent, calculated on the liquid, is 0.5-6.0 wt.%.

The liquid based composition according to the present invention, besides liquid and gelling agents, can also comprise other components. Such additional components may be immiscible with the liquid in which the gelling agent is dissolved. Thus the present composition can contain two or more distinct phases. Although, in case the additional immiscible components account for the major part of the composition, it may be argued that such a product is not a liquid based composition, such a composition is nevertheless encompassed by the present application. Indeed the presence of a relatively mobile, gelling agent containing liquid, is also appreciated in such products containing at least two distinct phases. An example of a fluid composition according to the present invention containing a component which is immiscible with the liquid, is a composition comprising a continuous aqueous phase containing the gelling agent and a fat phase which is dispersed in said aqueous phase.

The fluid composition according to the present invention, due to its special rheology and provided the right ingredients are added thereto, can advantageously be utilized as a product for personal care including cosmetic products, such as skin cream, moisturizer lotion, hair gel, deodorant, anti-perspirant etc., or as a pharmaceutical product such as ointment. The present fluid composition usually forms the bulk of such pharmaceutical products or products of personal care.

The present composition can beneficially be employed in both relatively viscous systems such as ointments and skin creams and less viscous systems such as deodorants. When utilized in ointments and skin creams the present composition offers the advantage that an appropriate rheology can be obtained without the need of using fatty emulsions which give a persisting greasy skin feel.

When used in, for instance, deodorants, the present composition offers the advantage that a viscous fluid composition is obtained by the inclusion of natural polymers, rather than the synthetic polymers normally used. The known inclusion of gelling polysaccharides in deodorants has the disadvantage that a more or less rigid gel is formed, hampering discharge from the container the deodorant is held in.

Examples of substances that may suitably be included in the pharmaceutical products and products for personal care according to the present invention are:

(i) Perfumes, which give rise to an olfactory response in the form of a fragrance, and deodorant perfumes, such as those described in US patent 4,278,658 (Lever Brothers & Co) which in addition to providing a fragrance response, can also reduce body malodour;

(ii) Skin coolants, such as menthol, menthyl lactate, menthyl pyrrolidone carboxylate N-ethyl-p-menthane-3-carboxamide and other derivatives of menthol, which give rise to a tactile response in the form of a cooling sensation on the skin; and

(iii) Emollients such as isopropylmyristate, silicone oils, mineral oils and vegetable oils which give rise to a tactile response in the form of an increase in skin lubricity.

(iv) Deodorants other than perfumes, whose function is to reduce the level of or eliminate microflora at the skin surface, especially those responsible for the development of body malodour. Precursors of deodorants other than perfume can also be employed.

(v) Antiperspirant actives, whose function is to reduce or eliminate the appearance of perspiration at the skin surface.

(vi) Anticholinergic actives, whose function is to reduce or eliminate the generation of perspiration before it reaches the skin surface, examples of which are scopolamine derivatives, such as scopolamine hydrobromide and esters thereof, such as benzoyl scopolamine hydrobromide.

(vii) Emulsifiers

Emulsifiers may be nonionic, anionic or cationic.
Examples of suitable emulsifiers are:

|  | HLB value |
|---|---|
| BRIJ 72 (Polyoxyethylene-2-stearyl ether) | 4.9 |
| BRIJ 52 (Polyoxyethylene-2-cetyl ether) | 5.3 |

(viii) Activity Enhancer

The composition according to the invention can also optionally comprise an activity enhancer, which can be chosen from a wide variety of molecules. Particular classes of activity enhancers include penetration enhancers and cationic polymers. Reference is made to co-pending European patent application No. 89304841.3 in which various suitable activity enhancers are mentioned.

(ix) Sunscreens

The emulsion according to the invention can also optionally comprise one or more sunscreen active ingredients.

The amount of the sunscreen active ingredient that can optionally be employed in accordance with the invention, as a therapeutically effective amount, will normally be from 0.01 to 10%, preferably from 0.1 to 5% and most preferably from 1 to 5% by weight of the composition.

In addition to the above ingredients conventionally used in products for personal care, the composition according to the invention can optionally comprise ingredients such as a colourant, preservative, antioxidant, in amounts which are conventional in the cosmetics, pharmaceutical etc.

The present fluid gelling agent containing composition, when having an appropriate rheology and containing particular ingredients such as flavouring, colouring, preservative, fat, etc., can suitably constitute a food product such as a spread, in particular a low calorie spread.

The composition according to the invention can contain various other ingredients such as colouring agent, flavouring, fat, salt, anti-oxidant, emulsifier etc. Preferably the composition according to the invention the composition is edible and contains, in combination, at least 90 wt.% of water and/or fat. Most preferably the present composition is water-continuous and contains from 5 to 30 wt.% fat.

An important advantage of the present composition as compared to the fluid compositions described in copending application EP-A 89 20 2066 is that they can be obtained in a form that is stable to high temperature treatment. Accordingly in a preferred embodiment of the present invention the composition is stable to pasteurization conditions.

Yet another aspect of the present invention is a process for preparing a spread containing from 5 to 60 wt.% of a continuous fat phase, including the admixture of fat with the present fluid composition. The use of the present fluid composition as the dispersed aqueous phase in fat-continuous spreads offers the advantage that, in particular in the preparation of spreads containing less than 45 wt.% fat, a fat-continuous product is obtained relatively easily and the risk of in-line re-inversion is reduced substantially. Also the use of the fluid composition allows the inclusion of destabilizing ingredients as these ingredients are 'captured' in the microgels. Most preferably the fluid composition is used in the preparation of spreads containing 15-30 wt.% of a continuous fat phase.

The invention is further illustrated by means of the following examples:

Example 1

Two aqueous compositions of the following formulation were prepared:

| Composition A | wt.% |
|---|---|
| Sodium alginate (Manucol DM $^{TM}$) | 2.0 |
| Sodium chloride | 1.7 |
| Potassium sorbate | 0.13 |
| Water to 100% | |

Composition B

| | |
|---|---|
| Calcium chloride (CaCl₂; anhydrous) | 1.32 |
| Sodium chloride | 1.7 |
| Potassium sorbate | 0.13 |
| Water to 100 % | |

The above aqueous compositions were both titrated to pH 5.5 with lactic acid and then placed in separate holding tanks at 70°C. The compositions A and B were mixed hot in the ratio 2:1 (alginate to calcium chloride) in a high speed C-unit. Thus the alginate level obtained was 1.33 wt.%. The molar ratio of alginate to calcium chloride (assuming the mol weight for alginate repeat unit to be 384) was roughly 1:1 with a slight excess of $Ca^{2+}$ ions.

The two aqueous compositions were mixed in the high speed C-unit by passing the alginate solution through said C-unit and introducing the $Ca^{2+}$ solution in the centre of the unit. The combined solutions upon leaving the C-unit are directly fed to a scraped surface heat exchanger (A-unit). The processing conditions employed in the C-unit and A-unit were as follows:

| | inlet temperature | jacket temperature | outlet temperature | r.p.m. |
|---|---|---|---|---|
| C-unit | 42°C [1] 54°C [2] | 74°C | 69°C | 1000 |
| A-unit | 69°C | 5°C | 15°C | 1400 |

[1] CaCl₂ solution
[2] Alginate solution

The product obtained at a throughput of 62g/min. was a pourable liquid and can suitably be used as an aqueous phase in spreads due to its liquid nature. The product contained small pieces of calcium alginate gel suspended in what was predominantly a sodium chloride solution.

The sheared alginate suspension was stable to pasteurization temperatures (above 80°C) and addition of further calcium ions had no effect. The suspension was particle sized by means of a Quantimet Image analyser. The mean particle size was measured to be about 10 micrometer.

The pourable liquid was viewed under a microscope at a magnification of x250. A photograph of the image obtained is represented in figure 1A. The pourable liquid was subsequently diluted by a factor 10 with deionised water after which a sample was taken and viewed under a microscope at magnification x250. A photograph of the image is represented in figure 1B.

The viscosity of the sheared alginate suspension at different shear rates and temperatures was measured and compared with the viscosity measured for unsheared alginate gels having the same composition but containing 1.5% respectively 1.0% sodium alginate by weight of the aqueous phase. The viscometer used to determine the viscosities were a Haake viscometer for low shear and a Ferranti Shirley viscometer for high shear viscosities.

The following data were obtained:

| Low Shear Viscosity at 1100 s⁻¹ | 10°C | 25°C | 30°C | 35°C | 40°C | 45°C |
|---|---|---|---|---|---|---|
| Sheared alginate | 112 | 100 | 93 | 85 | 81 | 77 |
| 1.5% alginate | 530 | 466 | 409 | 362 | 324 | 285 |
| 1.0% alginate | 166 | 146 | 123 | 112 | 100 | 81 |

| High Shear Viscosity at 10°C and— | 1709 $s^{-1}$ | 3000 $s^{-1}$ | 5000 $s^{-1}$ | 10000 $s^{-1}$ | 17090 $s^{-1}$ |
|---|---|---|---|---|---|
| Sheared alginate | 38 | 26 | 22 | 18 | 18 |
| 1.5% alginate | 151 | 98 | 73 | 49 | 37 |
| 1.0% alginate | 110 | 68 | 54 | 41 | 32 |

From these data it is evident that the sheared alginate suspension, containing 1.33 wt.% alginate is substantially less viscous that alginate gels of the same composition, obtained under quiescent conditions. The differences are most manifest at low temperatures (e.g. 10°C) and relatively low shear rates.

Example 2

A low fat spread was prepared from the above sheared alginate suspension by mixing it with a fat phase in a weight ratio of 40:60. The fat phase used had the following composition:

| Fat phase | % by weight of product |
|---|---|
| Fat blend * | 39.59 |
| Monoglyceride (Hymono 8903) | 0.20 |
| Lecithin (Bolec Z) | 0.20 |
| Beta carotene | 0.01 |

* Consisting of 50% soybean oil, 13% coconut oil, 17% soybean oil hardened to 41°C and 20% of soybean oil hardened to 33°C

The spread was prepared via an inversion process, using a sequence of 5 processing units (C-A-C-A-C). The exact processing conditions employed were as follows:

| | inlet temperature | jacket temperature | outlet temperature | r.p.m. |
|---|---|---|---|---|
| C-unit | 37°C [1] 44°C [2] | 25°C | 28°C | 1000 |
| A-unit | 28°C | 10°C | 16°C | 1000 |
| C-unit | 16°C | 11°C | 18°C | 1200 |
| A-unit | 18°C | 10°C | 16°C | 800 |
| C-unit | 16°C | 10°C | 17°C | 1200 |

A low fat spread was obtained at a throughput of 40.3 g/min. The product was found to spread smoothly and easily with no water loss. The volume weighted average aqueous droplet size, as measured by means of NMR, was found to be 10 micrometer.

The hardness of the product at 5° and 15°C, measured as the $C_5$- and $C_{15}$-value, was found to be 1070 and 380 g/cm$^3$ respectively. The method for determining the C-values is described in Journal of American Oil Chemists' Society 36 (1959), p. 345.

### Examples 3 and 4

Example 1 was repeated, with the exception that the Calcium chloride concentrations utilized were 0.66 and 2.64 wt.% respectively. The mean particle size of the fluid compositions so obtained, was measured to be about 10 microns.

The products contained small pieces of calcium alginate gel suspended in what was predominantly a sodium chloride solution. The sheared alginate suspensions were stable to pasteurization temperatures (above 80°C) and addition of further calcium ions had no effect.

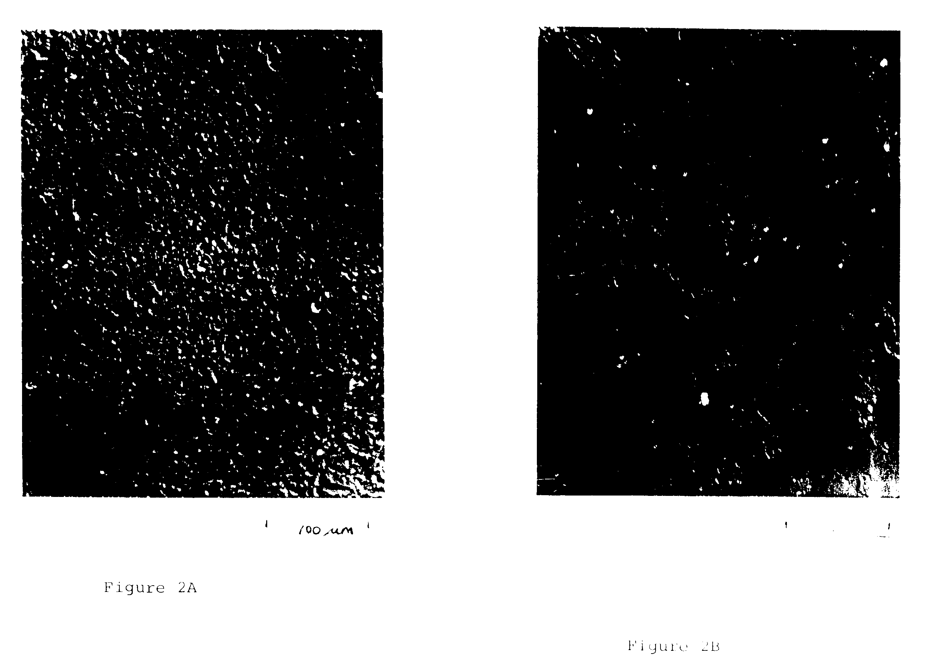

The pourable liquid obtained with 0.66 wt.% calcium chloride was viewed under a light microscope at a magnification of x250. A photograph of the image obtained is represented in figure 2A. The pourable liquid was subsequently diluted by a factor 10 with deionised water after which a sample was taken and viewed under a microscope at magnification x250. A photograph of the image is represented in figure 2B.

### Example 5

Example 1 was repeated with the exception that Composition A contained 3.0 wt.% sodium alginate and composition B contained 1.98 wt.% calcium chloride.

A pourable liquid, stable to pasteurization temperatures, was obtained. The addition of further calcium ions to the suspension had no effect. The mean particle size of the suspension was found to be about 20 micrometer.

### Example 6

Two aqueous compositions of the following formulation were prepared:

| Composition A | wt.% |
|---|---|
| Pectin (Obipektin Aer, Purple Ribbon "D" | 2.0 |
| Sodium chloride | 1.7 |
| Potassium sorbate | 0.13 |
| Water to 100% | |

| Composition B | |
|---|---|
| Calcium chloride ($CaCl_2$; anhydrous) | 1.32 |
| Sodium chloride | 1.7 |
| Potassium sorbate | 0.13 |
| Water to 100 % | |

Composition B was titrated to pH 5.5 with lactic acid. Composition A was titrated to pH 4.5 to minimize chain hydrolysis. The two compositions were placed in separate holding tanks at 75°C. The compositions A and B were mixed hot in the ratio 2:1 (pectin to calcium chloride) in a high speed C-unit. Thus the pectin level obtained was 1.33 wt.%.

The two aqueous compositions were mixed in the high speed C-unit by passing the alginate solution through said C-unit and introducing the $Ca^{2+}$ solution in the centre of the unit. The combined solutions upon leaving the C-unit are directly fed to a scraped surface heat exchanger (A-unit). The processing conditions employed in the C-unit and A-unit were as follows:

| | jacket temperature | outlet temperature | r.p.m. |
|---|---|---|---|
| C-unit | 75°C | 70°C | 1000 |
| A-unit | 5°C | 8°C | 1400 |

The product obtained at a throughput of 68g/min. was a pourable liquid and can suitably be used as an aqueous phase in spreads due to its liquid nature. The rheology of the pectin suspension was very much alike that of the alginate suspension described in Example 1.

8

Examples 7-12

Low fat spreads were prepared from the sheared alginate suspensions I, II and III, described in Examples 1, 3 (0.66 wt,% CaCl$_2$) and 5 respectively. The suspensions were mixed with two different fat phases A and B in a weight ratio of 40:60. The fat phases used had the following composition:

| Fat phase | % by weight of product |
|---|---|
| Fat blend | 39.59 |
| Monoglyceride (Hymono 8903) | 0.20 |
| Lecithin (Bolec Z) | 0.20 |
| Beta carotene | 0.01 |

The fat blend of fat phase A consisted of: 50% soybean oil, 13% coconut oil, 17% soybean oil hardened to a slip melting point of 41°C and 20% of soybean oil hardened to a slip melting point of 33°C

The fat blend of fat phase B consisted of: 35% rape seed oil hardened to a slip melting point of 32°C, 20% palm oil and 45% rape seed oil

The spreads were prepared via an inversion process, using a sequence of 5 processing units (C-A-C-A-C). The exact processing conditions employed were as follows:

| | inlet temperature | outlet temperature | r.p.m. |
|---|---|---|---|
| C-unit | | 36°C | 4500 |
| A-unit | 36°C | 14°C | 1000 |
| C-unit | 14°C | 17°C | 1300 |
| A-unit | 17°C | 13°C | 1000 |
| C-unit | 13°C | 14°C | 1000 |

Fat-continuous spreads were obtained at a throughput of 60 g/min.

| Alginate Suspension | Fat phase | Hardness at 5°C | Spreadability | Conductivity in S*10$^{-6}$ |
|---|---|---|---|---|
| II | B | 880 g/cm$^2$ | 2 | 0.37 |
| I | B | 800 | 2 | 0.83 |
| II | A | 950 | 4 | 0.0016 |
| I | A | 1100 | 2/3 | 0.0009 |
| III | B | 850 | 2 | 0.61 |
| III | A | 1000 | 2 | 0.06 |

The spreadability was evaluated by spreading the product onto a piece of paper and comparing the appearance of the product after spreading with 5 photographs showing products ranging from a spread having excellent spreading properties (no. 1) to a spread having unacceptable spreading properties (no. 5).

The volume weighted average aqueous droplet size for each of the 6 low fat spreads was found to be in the range of 1-10 micrometer.

## Claims

1. A process for preparing a fluid composition containing a chemically setting gelling agent, wherein a liquid containing the gelling agent is chemically set, whilst subjecting said liquid to sufficient shear to obtain a substantially less rigid composition than would have been obtained by chemically setting the liquid under quiescent conditions, wherein the composition comprises at least 75 wt% of the liquid and wherein either (a) the gelling agent comprises a gelling agent selected from the group consisting to pectin, iota-carrageenan, kappa-carrageenan, furcellaran and mixtures thereof, or (b) the gelling agent is alginate and the liquid containing the gelling agent in set at a temperature above 30°C.

2. Process according to claim 1, wherein the liquid is chemically set by allowing the gelling agent to form a salt with an effective cation.

3. Process according to claim 2, wherein the cation is selected from the group consisting of $Ca^{2+}$, $K^+$ and mixtures thereof.

4. Process according to claim 2 or 3, wherein the liquid is chemically set by combining two separate streams, one containing the gelling agent and another containing the cation.

5. Process according to claim 4, wherein the two streams are separately fed to an apparatus in which both streams are combined whilst applying and maintaining sufficient shear to obtain a substantially less rigid composition than would have been obtained by chemically setting the liquid under quiescent conditions.

6. Process according to any one of claims 1-5, wherein the liquid is subjected to sufficient shear to obtain a fluid composition in which the gelling agent is predominantly present as microgels having a mean equivalent diameter of less than 100 micrometer, preferably of less than 50 micrometer.

7. Process according to any one of claims 1-6, wherein the fluid composition is dried to a granular powder containing less than 50 wt%, preferably less than 25 wt% of the liquid.

8. Fluid composition containing a chemically setting gelling agent, at least part of which gelling agent has been chemically set, wherein the chemically set gelling agent is predominantly present as microgels having a mean equivalent diameter of less than 100 micrometer, preferably of less than 50 micrometer, and wherein either (a) the chemically setting gelling agent comprises a gelling agent selected from the group consisting of pectin, iota-carrageenan, kappa-carrageenan, furcellaran and mixtures thereof, and/or (b) the microgels are irregularly shaped.

9. Composition according to claim 8, wherein at least 2%, more preferably at least 10% of the number of microgels exceed 5 micrometer, using a Coulter Counter MultiSizer analysis.

10. Composition according to claim 8, wherein the composition contains at least 50 wt% of a liquid selected from the group consisting of water, ethanol iso-propanol and mixtures thereof.

11. Process for preparing a spread containing from 5 to 60 wt% fat, including the admixture of fat with the fluid composition according to any of claim 8-10.

## Patentansprüche

1. Verfahren zur Herstellung einer fließfähigen Zusammensetzung mit einem chemisch erstarrenden Geliermittel, wobei eine das Geliermittel enthaltende Flüssigkeit chemisch zum Erstarren gebracht wird, während die Flüssigkeit hinreichenden Scherkraften unterworfen wird, um eine weniger steife Zusammensetzung zu erhalten als man durch eine chemische Erstarrung der Flüssigkeit unter ruhigen Bedingungen erhalten würde, wobei die Zusammensetzung mindestens 75 Gew.-% Flüssigkeit umfaßt und wobei entweder (a) das Geliermittel ein Geliermittel umfaßt, ausgewählt aus der aus Pektin, Iota-Carrageenan, Kappa-Carrageenan, Furcellaran und deren Gemischen bestehenden Gruppe, oder (b) das Geliermittel Alginat ist und die das Geliermittel enthaltende Flüssigkeit bei einer Temperatur oberhalb 30°C zum Erstarren gebracht wird.

2. Verfahren nach Anspruch 1, dadurch gekennzeichnet, daß die Flüssigkeit dadurch chemisch zum Erstarren gebracht wird, daß man das Geliermittel mit einem wirksamen Kation ein Salz bilden läßt.

3. Verfahren nach Anspruch 2, dadurch gekennzeichnet, daß das Kation aus der aus $Ca^{2+}$, $K^+$ und Mischungen davon bestehenden Gruppe ausgewählt wird.

4. Verfahren nach den Ansprüchen 2 oder 3, dadurch gekennzeichnet, daß die Flüssigkeit chemisch zum Erstarren gebracht wird, indem zwei separate Ströme kombiniert werden, wobei der eine das Geliermittel und der andere das Kation enthält.

5. Verfahren nach Anspruch 4, dadurch gekennzeichnet, daß die zwei Ströme getrennt voneinander einer Vorrichtung zugeführt werden, in der beide Ströme kombiniert werden, während hinreichende Scherkräfte angewandt und aufrechterhalten werden, um eine im wesentlichen weniger steife Zusammensetzung zu erhalten als man durch ein chemisches Erstarren unter ruhigen Bedingungen erhalten würde.

6. Verfahren nach irgendeinem der Ansprüche 1 bis 5, dadurch gekennzeichnet, daß die Flüssigkeit hinreichenden Scherkräften unterworfen wird, um eine fließfähige Zusammensetzung zu erhalten, in der das Geliermittel vorwiegend in Form von Mikrogelen vorliegt, die einen mittleren Äquivalenzdurchmesser von weniger als 100 Mikrometern, vorzugsweise von weniger als 50 Mikrometern, haben.

7. Verfahren nach irgendeinem der Ansprüche 1 bis 6, dadurch gekennzeichnet, daß die fließfähige Zusammensetzung zu einem körnigen Pulver getrocknet wird, das weniger als 50 Gew.-%, vorzugsweise weniger als 25 Gew.-%, der Flüssigkeit enthält.

8. Fließfähige Zusammensetzung mit einem chemisch erstarrenden Geliermittel, wobei mindestens ein Teil des Geliermittels chemisch zum Erstarren gebracht wurde und wobei das chemisch erstarrte Geliermittel überwiegend in Form von Mikrogelen vorliegt mit einem mittleren Äquivalenzdurchmesser von weniger als 100 Mikrometern, vorzugsweise weniger als 50 Mikrometern, und wobei entweder (a) das chemisch erstarrende Geliermittel ein Geliermittel umfaßt, das aus der aus Pektin, Iota-Carrageenan, Kappa-Carrageenan, Furcellaran und deren Gemischen bestehenden Gruppe ausgewählt ist, und/oder (b) die Mikrogele unregelmäßig geformt sind.

9. Zusammensetzung nach Anspruch 8, dadurch gekennzeichnet, daß mindestens 2%, vorzugsweise mindestens 10% der Anzahl der Mikrogele 5 Mikrometer überschreiten, wobei die Coulter-Counter-MultiSizer-Analyse angewendet wird.

10. Zusammensetzung nach Anspruch 8, dadurch gekennzeichnet, daß die Zusammensetzung mindestens 50 Gew.-% einer Flüssigkeit enthält, die aus der aus Wasser, Ethanol, Isopropanol und deren Gemischen bestehenden Gruppe ausgewählt ist.

11. Verfahren zur Herstellung eines Brotaufstriches mit 5 bis 60 Gew.-% Fett, umfassend die Zumischung von Fett mit der fließfähigen Zusammensetzung entsprechend irgendeinem der Ansprüche 8 bis 10.

**Revendications**

1. Procédé de preparation d'une composition fluide contenant un agent gélifiant qui se fige chimiquement, dans lequel un liquide contenant l'agent gélifiant se fige chimiquement, tandis que ledit liquide est soumis à un cisaillement suffisant pour donner une composition sensiblement moins rigide que celle que l'on aurait obtenu en figeant chimiquement le liquide dans des conditions de repos, dans lequel la composition comprend au moins 75% en poids du liquide et dans lequel soit (a) l'agent gélifiant comprend un agent gélifiant choisi dans le groupe constitué de la pectine, la iota-carraghénine, la kappa-carraghénine, la furcellarane et des mélanges de celles-ci, soit (b) l'agent gélifiant est un alginate et le liquide contenant l'agent gélifiant se fige à une température supérieure à 30°C.

2. Procédé selon la revendication 1, dans lequel le liquide se fige chimiquement grace à la formation d'un sel entre l'agent gélifiant et un cation efficace.

3. Procédé selon la revendication 2, dans lequel le cation est choisi dans le groupe constitué par $Ca^{2+}$, $K^+$

et des mélanges de ceux-ci.

4. Procédé selon la revendication 2 ou 3, dans lequel le liquide se fige chimiquement par combinaison de deux courants séparés, l'un contenant l'agent gélifiant et l'autre contenant le cation.

5. Procédé selon la revendication 4, dans lequel les deux courants sont chargés séparément dans un appareil dans lequel les deux courants sont combinés, pendant qu'un cisaillement suffisant est appliqué et maintenu pour donner une composition sensiblement moins rigide que celle que l'on aurait obtenu en figeant chimiquement le liquide dans des conditions de repos.

6. Procédé selon l'une quelconque dees revendications 1-5, dans lequel le liquide est soumis à un cisaillement suffisant pour donner une composition fluide dans laquelle l'agent gélifiant est présent de façon prédominante sous forme de microgels possédant un diamètre moyen équivalent inférieur à 100 μm, de préférence inférieur à 50 μm.

7. Procédé selon l'une quelconque des revendications 1-6, dans lequel la composition fluide est séchée pour donner une poudre granulaire contenant moins de 50% en poids, de préférence moins de 25% en poids, du liquide.

8. Composition fluide contenant un agent gélifiant se figeant chimiquement, dont une partie au moins s'est figée chimiquement, dans laquelle l'agent gélifiant figé chimiquement est présent de façon prédominante sous forme de microgels possédant un diamètre moyen équivalent inférieur à 100 μm, de préférence inférieur à 50 μm, et dans laquelle (a) l'agent gélifiant qui se fige chimiquement comprend un agent gélifiant choisi dans le groupe constitué de la pectine, la iota-carraghénine, la kappa-carraghénine, la furcellarane et des mélanges de celles-ci, et/ou (b) les microgels sont de forme irrégulière.

9. Composition selon la revendication 8, dans laquelle au moins 2%, mieux encore au moins 10%, du nombre de microgels dépasse 5 μm, l'analyse étant faite à l'aide d'un compteur Coulter MultiSizer.

10. Composition selon la revendication 8, dans laquelle la composition contient au moins 50% en poids d'un liquide choisi dans le groupe constitué par l'eau, l'éthanol, l'isopropanol et des mélanges de ceux-ci.

11. Procédé de préparation d'un produit à tartiner contenant de 5 à 60% en poids de matière grasse, comprenant le mélange de la matière grasse avec la composition fluide selon l'une quelconque des revendications 8-10.

EP 0 432 835 B1

Figure 1B

Figure 1A

13

Figure 2B

100 μm

Figure 2A